# EUROPÄISCHE PATENTANMELDUNG

(11) **EP 2 278 376 A2**
(43) Veröffentlichungstag der Anmeldung: **26.01.2011**
(21) Anmeldenummer: 10186203.5
(22) Anmeldetag: 25.01.2006
(51) Int. Cl.: G02B 21/08, G02B 21/22, A61B 19/00, G02B 21/00

(54) **Operationsmikroskop mit zwei Beleuchtungsvorrichtungen**

(30) Priorität: 09.02.2005 DE 102005005984
(62) Teilanmeldung aus: 06100806.6
(71) Anmelder: Leica Instruments (Singapore) Pte. Ltd., 608924 Singapore (SG)
(72) Erfinder: Spink, Roger, 9442, Berneck (CH)
(74) Vertreter: Patentbüro Paul Rosenich AG

(57) **Zusammenfassung**

Die Erfindung betrifft ein Fluoreszenz-Operations Steromikroskop mit einer Weisslicht-Beleuchtungsquelle (20), deren Licht in einem regulierbaren spektralen Bereich liegt. Dieser Beleuchtungsvorrichtung ist mindestens eine weitere Beleuchtungsvorrichtung (3), deren Licht in davon verschiedenen, regulierbaren Spektralbereichen liegt, frei zuschaltbar.

## Beschreibung

Die Erfindung bezieht sich auf ein Operations-Stereomikroskop mit Fluoreszenzbetriebsmodus mit einer Beleuchtungsquelle, deren Licht vorbestimmten spektralen Umfangs (Weisslicht) mittels einer Optikeinrichtung gegen ein zu betrachtendes Objekt richtbar ist. Wahlweise ist das Licht mindestens einer weiteren Beleuchtung in einem anderen Spektralbereich zuschaltbar.

Die Fluoreszenz ist eine hinreichend bekannte Methode, die mit Hilfe abgestimmter Filter ein definiertes Anregungsspektrum einem Objekt zuführt und die vom Objekt abgestrahlte Signalantwort spektral vom Anregungslicht trennt und der Beobachtung und Analyse zuführt. So sind etwa im klinischen Bereich viele Applikationen bekannt, die chirurgische Eingriffe unterstützen und über die ausgesendete Fluoreszenz das zu resizierende Gewebe markieren. Ein besonderes Beispiel für die Anwendung einer solchen Methode mit in ein Mikroskop integrierten Fluoreszenzeinrichtungen sind Operationsmikroskope für die Neurochirurgie, die unter Verwendung photodynamischer Medikamente, welche z.B. unter den Namen ALA (Amino Levulinic Acid = Amino-Lävulinsäure) oder mTHPC (meso-Tetra-HydroxyPhenyl-Chlorin) bekannt sind, eine totalere Operation bestimmter Tumore zulassen.

Eine andere Anwendung betrifft etwa die Infrarot-Angiographie, bei der mit Licht aus dem NIR-(nahen Infrarot-)Bereich zur Anregung verwendet wird, um dann das Objekt im langwelligeren Spektralbereich zu beobachten. Andere Anwendungen machen von (nicht sichtbarem) Ultraviolett Gebrauch. Andere Spektralbereiche zwischen Ultraviolett zu Blaulicht und von dort zu Rot- und bis zum fernen Infrarot sind ebenfalls möglich.

Dort, wo ein Gewebe bzw. ein Objekt, angeregt werden soll, ist die genügende Intensität des Anregungsspektrums von wesentlicher Bedeutung.

Wenn etwa mit blauem Anregungslicht im Bereich von 380 - 420 nm gearbeitet wird, wird man je nach verwendetem Fluoreszenz-Wirkstoff ein bestimmtes Fluoreszenzsignal (z.B. bei ALA 635 nm) erhalten. Dazu werden im Allgemeinen Xenon-Lichtquellen von 300 W eingesetzt, die sowohl das normale mikroskopische Weisslicht, als auch das für die Fluoreszenz notwendige Blaulicht bereitstellen, nämlich Letzteres durch Filterung sowie Optimierung des Spektralbereichs von 380-420 nm durch gezielte Auswahl des Xenon-Brenners. Analoges gilt natürlich für andere Spektralbereiche. Beispiele für solche bekannten Mikroskope bzw. Operationsmikroskope finden sich etwa in der US-A-6,510,338 oder der DE-A-195 48 913. Dort wird das Licht der Beleuchtungseinrichtung über Lichtwellenleiter und andere optische Einrichtungen dem zu beobachtenden Objekt zugeführt.

Problematisch an diesen bekannten Mikroskopen ist, dass hier bei der Auswahl der Beleuchtungsquelle ein Kompromiss geschlossen werden muss, der letzten Endes dazu führt, dass die Weisslichtqualität für die Beobachtung nicht optimiert sein kann, anderseits gerade bei erhöhtem und optimierten Blaulichtanteil andere Spektralbereiche unterproportional vertreten sind und dann zu Farbstichen in der Standard-Weisslichtsituation führen. Zwar ist eine Korrektur des Farbstichs durch Filter theoretisch möglich, führt aber dann auch zur Reduktion der Intensität. Anderseits ist eine farblich richtige Beobachtung eines Operationsbereiches schon aus diagnostischen Gründen wichtig. Eine Steigerung der Intensität ist aber aufgrund der begrenzten Apertur der mikroskopischen Beleuchtungsoptik und anderer Effekte über eine Erhöhung der Lampenleistung nicht möglich.

Der vorliegenden Erfindung liegt deshalb die Aufgabe zugrunde, ein Mikroskop der eingangs genannten Art derart auszubilden, dass - trotz der gegebenen Einschränkung durch die Apertur der Beleuchtungsoptik - eine Intensitätserhöhung der Beleuchtung sowohl im Weisslichtbetrieb, wie auch im Anregungsmodus möglich ist. Eine weitere Aufgabe der Erfindung liegt darin, die Weisslichtqualität zu verbessern.

Zur Lösung dieser Aufgaben wird erfindungsgemäss vorgeschlagen, dass die Optikeinrichtung mit mindestens einem Anschluss für eine weitere Beleuchtungsquelle versehen ist. Erfindungsgemäss soll also mindestens ein Anschluss für eine weitere Beleuchtungsquelle vorgesehen sein, wobei es dann ohne Weiteres möglich ist, die eine Beleuchtungsquelle mit dem vorbestimmten Spektralbereich als Weisslichtquelle zu optimieren, die andere hingegen mit demjenigen Spektralbereich optimiert einzusetzen, welcher für eine bestimmte Anwendung gerade erforderlich und zweckmässig ist. Denkbar wäre sogar die Anordnung mehrerer Anschlüsse für mehr als zwei Beleuchtungsquellen, die dann durch entsprechende optische und/oder elektrische Schalteinrichtungen zur Wirkung gebracht werden können.

Es ist im Rahmen der Erfindung aber auch möglich, die jeweilige weitere Beleuchtungsquelle in das Mikroskop zu integrieren, wobei die Ausbildung so getroffen werden kann, dass das Mikroskop dann die mindestens eine weitere Beleuchtungseinrichtung enthält, und dass diese weitere Beleuchtungseinrichtung einen vom vorbestimmten spektralen Umfang abweichenden spektralen Umfang besitzt.

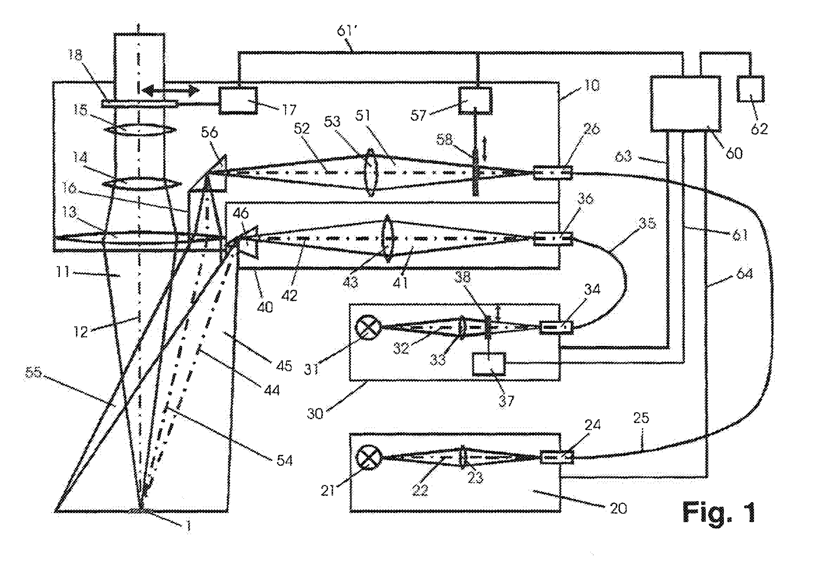

Weitere Ausgestaltungen der Erfindung ergeben sich aus der nachfolgenden symbolischen und beispielhaften Beschreibung an Hand der Figuren sowie aus den abhängigen Ansprüchen, wobei die Bezugszeichenliste Bestandteil der Offenbarung ist. In der Beschreibung werden die Figuren zusammenhängend und übergreifend beschrieben. Gleiche Bezugszeichen bedeuten gleiche Bauteile, Bezugszeichen einer unterschiedlichen Dekade (10, 20, 30, etc.) geben funktionsgleiche oder ähnliche Bauteile an. Es zeigen dabei die
Fig. 1-4 : je eine Ausführungsvariante der Erfindung in Form einer schematischen Darstellung.

Gemäss Fig. 1 soll ein Objekt 1 mittels eines Mikroskops betrachtet werden, das einen schematisch angedeuteten Mikroskopkörper 10 in an sich bekannter Bauweise besitzt. An diesem Mikroskopkörper 10 ist eine Optik zur Erzeugung eines Beobachtungsstrahlengangs 11 entlang einer optischen Achse 12 in an sich bekannter Weise montiert, die ein, lediglich als Linse angedeutetes Hauptobjektiv 13, gegebenenfalls zwei Linsen oder -gruppen 14, 15 einer Zoomoptik, ein mittels einer elektromechanischen Bewegungseinrichtung 17, wie eines Elektromagneten, eines Läufermotors oder einem ähnlichen Motor (in weitestem Sinn), im Bedarfsfall in den Beobachtungsstrahlengang 11 einschwenk- oder einschiebbaren Beobachtungs-(Offenbarung: Absatz 16 Zeile 33-34) Filter 18 und gegebenenfalls ein an der Oberseite des Beobachtungsstrahlengangs 11 ein (nicht dargestelltes) Okular trägt. Es versteht sich, dass im Falle eines Stereomikroskops, zwei solcher Beobachtungsstrahlengänge vorgesehen sind.

Um das Objekt 1 auch entsprechend zu beleuchten, sind erfindungsgemäss (mindestens) zwei Licht- bzw. Beleuchtungsquellen 20, 30 vorgesehen, die über je einen Beleuchtungsstrahlengang 41 bzw. 51 und eine Umlenkspiegelfläche, vorzugsweise in Form einer Spiegelfläche an einem Beleuchtungsprisma 46 bzw. 56 je einen Beleuchtungsstrahlengang 45 bzw. 55 entlang einer zugehörigen optischen Achse 44 bzw. 54 auf das zu beobachtende Objekt 1 werfen. Durch die Erfindung ist es möglich, die Beleuchtungsquellen optimal den Erfordernissen anzupassen. Beispielsweise kann für Weisslicht (also für die Lichtquelle 20) besonders eine Xenonlampe eingesetzt werden. Für Lichtwellenlängen im Bereiche um 400 nm (Blaulicht) wird sich eine Quecksilberdampflampe empfehlen, und für Infrarot kommen die verschiedensten Lichtquellen in Frage

Jeder dieser Beleuchtungsstrahlengänge von der Lichtquelle 20 bzw. 30 zum Objekt 1 umfasst jeweils einen Beleuchtungskörper 21 bzw. 31, der Licht (hier sei unter "Licht" sichtbares und unsichtbares Licht, also allgemein elektromagnetische Strahlung verstanden) entlang einer optischen Achse 22 bzw. 32 über eine Beleuchtungsoptik 23 bzw. 33 zum Eintritt 24 bzw. 34 eines Lichtwellenleiters 25 bzw. 35 sendet, der das Licht zu einem Lichtaustritt 26 bzw. 36 leitet. In der Zeichnung ist der Lichtwellenleiter 25 bzw. 35 gekrümmt, d.h. als Lichtleitfaserbündel dargestellt, doch ist die Erfindung keineswegs darauf beschränkt, vielmehr könnte die jeweilige Beleuchtungsquelle 20 bzw. 30 durchaus auch so angebracht sein, dass ihre optische Achse 22 bzw. 32 mit einer am Mikroskopkörper 10 weiterführenden optischen Achse 42 bzw. 52 je eines Beleuchtungsstrahlengangs 41 bzw. 51 zusammenfällt. Auch andere Arten von Lichtwellenleitern sind im Rahmen der Erfindung durchaus denkbar und möglich, wenngleich die dargestellte Ausführung mit Lichtwellenleitern 25 bzw. 35, oder wenigstens einem davon, bevorzugt ist. Für gewöhnlich ist etwa eine Standard-Beleuchtungsquelle 20 bereits in das Stativ eines Operationsmikroskops eingebaut. Für die erfindungsgemäss vorgesehene zweite Lichtquelle 30, die allenfalls als externes Gerät vorliegen kann, braucht an sich der Lichtleiteraustritt 36 nur als Anschluss für einen nachträglich anzukuppelnden Lichtwellenleiter 35 ausgebildet sein. Anschlüsse für Lichtwellenleiter sind an sich bekannt, weshalb hier auf eine detaillierte Erörterung verzichtet wird. Zweckmässig ist dieser Anschluss 34 dann an einem eigenen, allenfalls an ein bestehendes Mikroskop anbaubaren Beleuchtungsmodul 40 mit den entsprechenden Optikteilen 43 und 46 angebracht.

Wie bereits angedeutet, führt die Austrittsseite 26 bzw. 36 des jeweiligen Lichtwellenleiters 25 bzw. 35 direkt zu einem optischen System entlang der optischen Achsen 42 und 52 von Beleuchtungsstrahlengängen 41 bzw. 51, an welchen Achsen je eine Beleuchtungssammellinse 43 bzw. 53 angeordnet ist. Natürlich kann diese Sammellinse 43 bzw. 53 aus mehreren Einzellinsen zusammengesetzt sein.

Schliesslich treffen die beiden optischen Achsen 42, 52 auf die Spiegelprismen 46 und 56, um in den schon erwähnten Beleuchtungsstrahlengang 45 bzw. 55 mit den optischen Achsen 44 und 54 umgelenkt zu werden. Diese Beleuchtungsstrahlengänge 45, 55 befinden sich nun nahe des Beobachtungsstrahlengangs 11, weshalb vorteilhaft dazwischen eine abdeckende Lichtblende 16 vorgesehen ist.

Im Betrieb wird nun, z.B. über einen mit einem Steuer- oder Kontrollgerät 60 verbundenen Schalter 62 (Hand- oder Fussschalter, Tastenfeld oder Sprachkontrolle, etc.) und über Leitungen 63 bzw. 64 jeweils eine der Beleuchtungsquellen 20 bzw. 30 eingeschaltet, um das Objekt 1 beispielsweise mit Weisslicht oder mit Blaulicht anzustrahlen. Soll auf eine Anregungswellenlänge bzw. -spektrum umgeschaltet werden, dann werden Filter 38 bzw. 58 für die Anregung bzw. 18 für die Beobachtung in den jeweiligen Strahlengang 11, 41 bzw. 51 gebracht. Dazu sind im Wesentlichen ähnliche elektromechanische Bewegungseinrichtungen 37 bzw. 57 vorgesehen wie oben anhand der Einrichtung 17 bereits beschrieben wurde. Alle diese Einrichtungen 17, 37 und 57 werden von der Kontrolleinrichtung 60 bzw. dem Schalter 62 über die Signalverbindung 61, 61' gesteuert, und zwar so, dass die Bewegung der Anregungsfilter 38, 58, vorteilhaft aber auch des Beobachtungsfilters 18, synchron erfolgt. Das bedeutet, dass diese Filter im Anregungsmodus gemeinsam in den jeweiligen Strahlengang bewegt und im Weisslichtmodus auch synchron aus dem Strahlengang bewegt werden. Dabei kann das Steuergerät als Hardware oder Software einen Endschalter mit Rückmeldung beinhalten, der verhindert, dass das Objekt ungewollt gleichzeitig mit Weisslicht und Anregungslicht beleuchtet wird.

Was in Fig. 1 als ein einziges Filter 38 dargestellt ist, kann (und dies betrifft auch die anderen Filter 18 und 58) mehrere, hintereinander angeordnete und wahlweise einschiebbare Filter umfassen. Alternativ (oder zusätzlich für den wahlweisen Einsatz) ist ein Filter für den Weisslichtmodus vorgesehen, durch welches das Spektrum der Lichtquelle 31 korrigiert wird. Schliesslich ist ein Anregungsfilter vorgesehen, das nur dann einschwenkt, um ausschliesslich die Anregungswellenlänge passieren zu lassen. Im Objektfeld kann dann die Beleuchtungsenergie beider Lichtquellen 21 bzw. 31 mit der gewünschten Anregungswellenlänge (bei eingeschwenktem Filter 58) zur Verfügung stehen, so dass die Intensität insgesamt erhöht wird. Allerdings kann der Schalter 62 auch dafür verwendet werden, eine der Lichtquellen 20 oder 30 abzuschalten, wenn für eine Anwendung zusätzliches Licht unerwünscht ist. Insbesondere für mehrere verschiedene - aber auch identische, synchrone - Anregungs- und/oder Beobachtungswellenlängen können auch Filterwechsler mit mehreren Filtersätzen vorgesehen werden, obwohl es die vorliegende Erfindung auch gestattet, mehrere Anschlüsse (vgl. den Anschluss 36) für mehrere Lichtquellen unterschiedlichen Anregungs-Spektralbereichs vorzusehen.

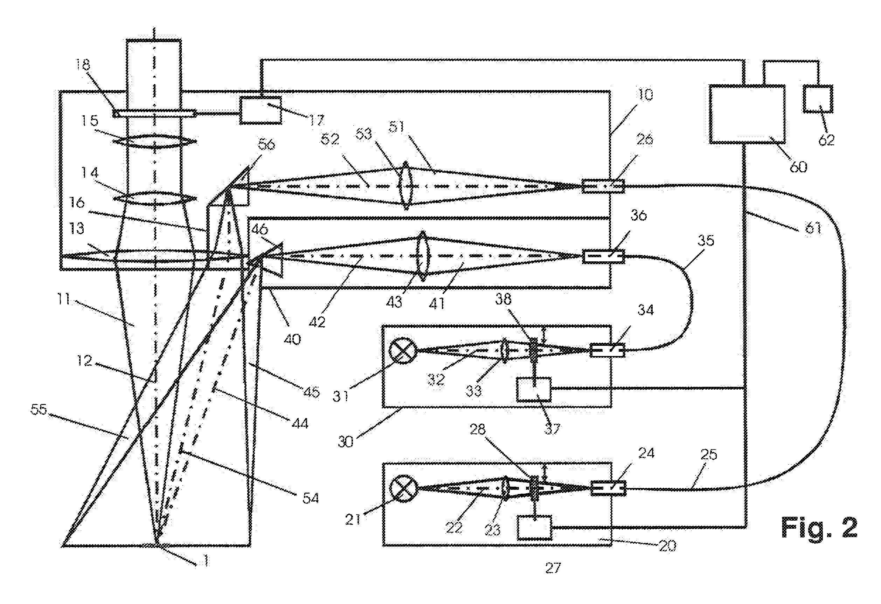

Das Ausführungsbeispiel der Fig. 2, in dem die Leitungen 61', 63 und 64 nicht dargestellt sind, unterscheidet sich von dem der Fig. 1 im Wesentlichen dadurch, dass das einzuschwenkende Anregungsfilter 58 (Fig. 1) im Mikroskopkörper 10 weggelassen und stattdessen ein Anregungsfilter 28 in die Lichtquelle 20 eingebaut ist, so dass der elektromechanische Aufwand im Mikroskopkörper 10 vermieden bzw. verringert ist. Dies ist also aus baulichen Gründen bevorzugt, zumal diese Ausführungsform auf die Funktion keine irgendwie nachteilige Auswirkung hat.

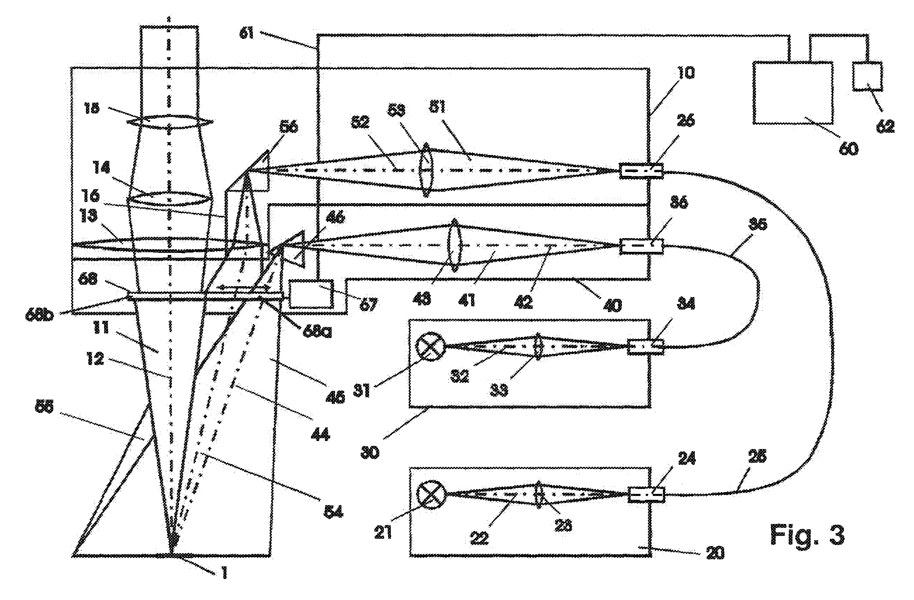

Auch die Ausführungsform nach Fig. 3 unterscheidet sich von den vorigen Ausführungsbeispielen durch die Unterbringung der Filter. Hier ist die Ausgangsleitung 61 des Steuergeräts 60 mit einer elektromechanischen Bewegungseinrichtung bzw. einem zentralen Motor 67 im Mikroskopkörper 10 verbunden, der einen Filtersatz 68 sowohl im Beobachtungsstrahlengang 11 als auch im Beleuchtungsstrahlengang 45 bzw. 55 derart verstellt, dass gleichzeitig das Anregungsfilter 68a für die erste und zweite Lichtquelle 20 bzw. 30 in den Beleuchtungsstrahlengängen 45 und 55 und das Beobachtungsfilter 68b im Beobachtungsstrahlengang 11 wirksam werden (oder aus diesen Strahlengängen entfernt werden). Der Aufwand an elektromechanischen Bewegungseinrichtungen ist bei dieser Ausführung deutlich gesenkt, wenngleich in Kauf zu nehmen ist, dass die Einrichtung im Mikroskopkörper 10 untergebracht werden muss. Welche der Ausführungen daher bevorzugt ist, insbesondere welche von denjenigen der Fig. 2 oder Fig. 3, wird von den jeweiligen Anwendungen und den baulichen Gegebenheiten abhängen. Es sei jedoch erwähnt, dass eine solche Ausführungsform es mit sich bringt, dass der Filtersatz 68 (und damit auch die elektromechanische Bewegungseinrichtung 67) relativ nahe an den Prismen 46 und 56 anzubringen sein werden, weil dort die Beleuchtungsstrahlaufweitung noch relativ gering ist. Anderseits ist in diesem Bereich die Aufweitung des Beobachtungsstrahlengangs 11 relativ gross, so dass man hier einen Kompromiss eingehen muss.

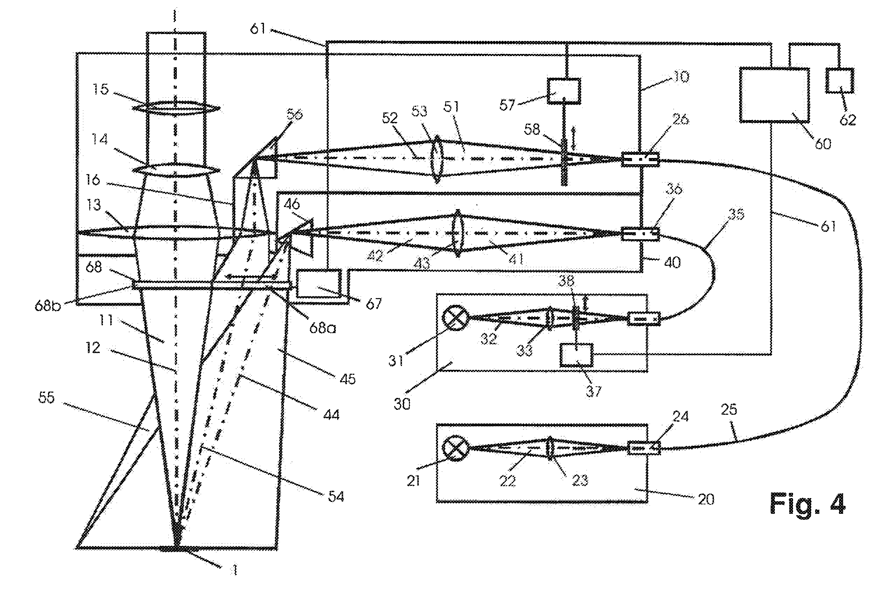

Die Ausführungsform nach Fig. 4 zeigt eine Kombination der Ausführungen nach Fig. 3 mit derjenigen der Fig. 1. Sie erlaubt es also, gegebenenfalls unterschiedliche Beleuchtungs- bzw. Anregungsfilter 68, 58 und/oder 38 zur Wirkung zu bringen, wofür das Steuergerät 60 mit entsprechenden Steuerleitungen bzw. Signalbussen 61, 61' ausgerüstet ist. Damit lässt sich diese Ausführungsform natürlich für die unterschiedlichsten Anwendungen einsetzen.

Im Rahmen der Erfindung sind zahlreiche Varianten möglich. Erfindungsgemäss ist vorgesehen, wenn für jedes der jeweils vorhandenen Filter 18, 28, 38, 58, 68 eine eigene elektromechanische Bewegungseinrichtung (also im weitesten Sinne ein "Motor" in seiner Bedeutung als "Beweger") vorgesehen ist.

Entscheidend sind erfindungsgemäss die Variationsmöglichkeiten, die sich aus der Kombination zweier regulierbaren Beleuchtungseinrichtungen ergeben. Demzufolge liegt auch eine Ausgestaltungsvariante eines Stereomikroskops im Rahmen der Erfindung, die eine herkömmliche Mikroskopbeleuchtung plus dem regulierbaren Paar der erfindungsgemässen Beleuchtungsvorrichtungen umfasst.
Nachfolgend der Text der ursprünglichen Ansprüche aus der Stammanmeldung, die im Rahmen dieser Teilanmeldung ausschliesslich aus Gründen der Übernahme ihres Offenbarungsinhaltes hier wieder gegeben werden und in der vorliegenden Form nicht beansprucht werden.
A. Stereomikroskop mit einer Beleuchtungsquelle (20), deren Licht im Betriebszustand in einem regulierbaren spektralen Bereich liegt und gegen ein zu betrachtendes Objekt (1) richtbar ist, **dadurch gekennzeichnet, dass** es mindestens eine weitere Beleuchtungseinrichtung (30) enthält, deren Licht im Betriebszustand ebenfalls regulierbar im gleichen spektralen Bereich liegt.
B. Stereomikroskop nach A, **dadurch gekennzeichnet, dass** der Spektralbereich der mindestens einen weiteren Beleuchtungseinrichtung (30) in einem von der ersten Beleuchtungseinrichtung (20) abweichenden Spektralbereich liegt.
C. Stereomikroskop nach B, **dadurch gekennzeichnet, dass** der von der ersten Beleuchtungseinrichtung (20) abweichende spektrale Bereich der mindestens einen weiteren Beleuchtungseinrichtung (30) im Blaulichtbereich von 380-420 nm liegt.
D. Stereomikroskop nach B, **dadurch gekennzeichnet, dass** der abweichende spektrale Bereich der mindestens einen weiteren Beleuchtungseinrichtung (30) im Ultraviolettbereich liegt.
E. Stereomikroskop nach B, **dadurch gekennzeichnet, dass** der abweichende spektrale Bereich der mindestens einen weiteren Beleuchtungseinrichtung (30) im Infrarotbereich liegt.
F. Stereomikroskop nach einem der vorhergehenden Punkte, **dadurch gekennzeichnet, dass** in mindestens eine der Beleuchtungsquellen (20 bzw. 30) wenigstens ein Beleuchtungs- und/oder Anregungsfilter (28 bzw. 38) in den Strahlengang des Beleuchtungskörpers (21 bzw. 31) bewegbar ist.
G. Stereomikroskop nach F, **dadurch gekennzeichnet, dass** das wenigstens eine Beleuchtungs- und/oder Anregungsfilter (28 bzw. 38) in zumindest einer der Beleuchtungsquellen (20 bzw. 30) mit Hilfe einer elektromechanischen Bewegungseinrichtung (27 bzw. 37) bewegbar ist.
H. Stereomikroskop nach Anspruch G, **dadurch gekennzeichnet, dass** die elektromechanische Bewegungseinrichtung (27 bzw. 37) in zumindest einer der Beleuchtungsquellen (20 bzw. 30) von einer zentralen Kontrolleinrichtung (60) gesteuert ist, die vorzugsweise zur Vermeidung von Fehlfunktionen mit einem Endschalter mit Rückmeldung versehen ist.
I. Stereomikroskop nach einem der vorhergehenden Punkte, **dadurch gekennzeichnet, dass** ein gemeinsamer Filtersatz (68) vorgesehen ist, der sowohl in den Beobachtungsstrahlengang (11) als auch in den Beleuchtungsstrahlengang bzw. die Beleuchtungsstrahlengänge (45 bzw. 55) bewegbar ist.
J. Stereomikroskop nach G, **dadurch gekennzeichnet, dass** eine gemeinsame elektromechanische Bewegungseinrichtung (67) zugeordnet ist.
K. Stereomikroskop nach einem der vorhergehenden Punkte, **dadurch gekennzeichnet, dass** Beleuchtungs- und/oder Anregungsfilter (28, 38, 58) für die Strahlengänge (22, 32, 41, 51, 45, 55) der zumindest zwei Beleuchtungsquellen (20, 30) vorgesehen sind, und dass eine gemeinsame Kontrolleinrichtung (60) zum synchronen Bewegen der Beleuchtungs-und/oder Anregungsfilter (28, 38, 58) vorgesehen ist.
L. Stereomikroskop nach K, **dadurch gekennzeichnet, dass** in den Beobachtungsstrahlengang (11) ein Beobachtungsfilter (18) mit Hilfe einer elektromechanischen Bewegungseinrichtung (17) bewegbar ist, und dass diese elektromechanische Bewegungseinrichtung (17) ebenfalls von der gemeinsamen Kontrolleinrichtung (60) gesteuert ist.
M. Stereomikroskop nach einem der vorhergehenden Punkte, **dadurch gekennzeichnet, dass** ein Weisslicht-Korrekturfilter in den Strahlengang der ersten Beleuchtungsquelle (20) bewegbar ist.
N. Stereomikroskop nach einem der vorhergehenden Punkte, **dadurch gekennzeichnet, dass** es als Operationsmikroskop ausgebildet ist.

### Bezugszeichenliste

- 1- :: Objekt
- 10 - :: Mikroskopkörper
- 11 - :: Beobachtungsstrahlengang
- 12 - :: Optische Achse von 11
- 13- :: Hauptobjektiv
- 14 - :: Linse oder Linsengruppe
- 15- :: Linse oder Linsengruppe
- 16- :: Lichtblende
- 17- :: Elektromechanische Bewegungseinrichtung
- 18- :: Filter
- 20- :: Lichtquelle 1, Beleuchtungseinrichtung
- 21 - :: Beleuchtungskörper
- 22 - :: Optische Achse der Lichtquelle
- 23- :: Beleuchtungsoptik
- 24- :: Lichtwellenleiter-Eintritt
- 25 - :: Lichtwellenleiter
- 26- :: Lichtwellenleiter-Austritt
- 27 - :: Elektromechanische Bewegungseinrichtung
- 28 - :: Beleuchtung-/Anregungsfilter
- 30- :: Lichtquelle 2
- 31- :: Beleuchtungskörper
- 32- :: Optische Achse der Lichtquelle
- 33- :: Beleuchtungsoptik
- 34 - :: Lichtwellenleiter Eintritt
- 35 - :: Lichtwellenleiter
- 36 - :: Lichtwellenleiter Austritt, Anschluss
- 37 - :: Elektromechanische Bewegungseinrichtung
- 38 - :: Beleuchtung-/Anregungsfilter
- 40 - :: Beleuchtungsmodul
- 41 - :: Beleuchtungsstrahlenbündel
- 42- :: Optische Achse des Beleuchtungsstrahlengangs
- 43- :: Beleuchtungssammellinse
- 44- :: Optische Achse des Beleuchtungsfeldbündels
- 45 - :: Beleuchtungsstrahlengang
- 46- :: Beleuchtungsprisma
- 51- :: Beleuchtungsstrahlengang
- 52- :: Optische Achse des Beleuchtungsstrahlengangs
- 53 - :: Beleuchtungssammellinse
- 54- :: Optische Achse des Beleuchtungsstrahlengangs 55
- 55 - :: Beleuchtungsstrahlengang
- 56 - :: Beleuchtungsprisma
- 57- :: Elektromechanische Bewegungseinrichtung
- 58- :: Beleuchtungs-/Anregungsfilter
- 60- :: Steuergerät/Kontrolleinrichtung
- 61- :: Steuersignal/Signalbus
- 62- :: Schalter
- 63- :: Steuersignal/Signalbus
- 64- :: Steuersignal/Signalbus
- 67- :: Elektromechanische Bewegungseinrichtung
- 68, a, b - :: Beleuchtungs- und Beobachtungsfilter

## Patentansprüche

1. Fluoreszenz-Operations-Stereomikroskop mit einer Weisslicht-Beleuchtungsquelle (20),
deren Licht im Betriebszustand in einem regulierbaren spektralen Bereich liegt und über einen einbringbaren Anregungsfilter für Fluoreszenzanregung verfügt und entlang voneinander unabhängigen Beleuchtungsstrahlengängen (42, 52, 44,54) gegen ein zu betrachtendes Objekt (1) richtbar ist,
**dadurch gekennzeichnet, dass** es mindestens eine weitere
zuschaltbare
Beleuchtungseinrichtung (30) enthält, deren Licht im Betriebszustand ebenfalls regulierbar in einem vom Weisslicht abweichenden
Spektralbereich liegt, **und dass** in mindestens eine der Beleuchtungsquellen (20 bzw. 30) wenigstens ein Anregungsfilter (28 bzw. 38) in den Strahlengang des Beleuchtungskörpers (21 bzw. 31) bewegbar ist, **und**
**dass** die Anregungsfilter (28, 38,58) in zumindest einer der Beleuchtungsquellen (20 bzw. 30) mit Hilfe einer elektromechanischen Bewegungseinrichtung (27 bzw. 37) bewegbar sind, **und**
**dass** die elektromechanische Bewegungseinrichtung (27 bzw. 37) in beiden Beleuchtungsquellen (20 bzw. 30) von einer zentralen Kontrolleinrichtung (60) gesteuert ist, die vorzugsweise zur Vermeidung von Fehlfunktionen mit einem Endschalter mit Rückmeldung versehen ist, **und**
**dass** die gemeinsame Kontrolleinrichtung (60) zum synchronen Bewegen der Anregungsfilter (28,38,58) vorgesehen ist, **und**
**dass** in den Beobachtungsstrahlengang (11) ein Beobachtungsfilter (18) mit Hilfe einer elektromechanischen Bewegungseinrichtung (17,67) bewegbar ist, **und**
**dass** diese elektromechanische Bewegungseinrichtung (17,67) ebenfalls von der gemeinsamen Kontrolleinrichtung (60) gesteuert ist, **und**
**dass** die beiden Beleuchtungsquellen (20,30) im Anregungsmodus ihr AnregungsLicht gleichzeitig (parallel) über je einen Beleuchtungsstrahlengang 41 bzw. 51 und eine Umlenkspiegelfläche, vorzugsweise in Form einer Spiegelfläche an einem Beleuchtungsprisma 46 bzw. 56 je einen Beleuchtungsstrahlengang 45 bzw. 55 entlang einer zugehörigen optischen Achse 44 bzw. 54 auf das zu beobachtende Objekt 1 werfen, **und**
**dass** die Anregungsfilter (28,38,58,68) im Weisslichtmodus synchron aus den Strahlengängen bewegt sind.

2. Stereomikroskop nach Anspruch 1, **dadurch gekennzeichnet, dass** der von der ersten Beleuchtungseinrichtung (20) abweichende spektrale Bereich der mindestens einen weiteren Beleuchtungseinrichtung (30) im Blaulichtbereich von 380-420 nm liegt.

3. Stereomikroskop nach Anspruch 1, **dadurch gekennzeichnet, dass** der abweichende spektrale Bereich der mindestens einen weiteren Beleuchtungseinrichtung (30) im Ultraviolettbereich liegt.

4. Stereomikroskop nach Anspruch 1, **dadurch gekennzeichnet, dass** der abweichende spektrale Bereich der mindestens einen weiteren Beleuchtungseinrichtung (30) im Infrarotbereich liegt.

5. Stereomikroskop nach einem der vorhergehenden Ansprüche, **dadurch gekennzeichnet, dass** alternativ oder zusätzlich
zu den einzelnen Anregungsfiltern (28,38,58) und Beobachtungsfilter (18) ein gemeinsamer Filtersatz (68, 68a)
vorgesehen ist, der sowohl in den Beobachtungsstrahlengang (11) als auch in den Beleuchtungsstrahlengang bzw. die Beleuchtungsstrahlengänge (45 bzw. 55) bewegbar ist.

6. Stereomikroskop nach Anspruch 5, **dadurch gekennzeichnet, dass** dem gemeinsamen Filtersatz (68,68a)
eine gemeinsame elektromechanische Bewegungseinrichtung (67) zugeordnet ist.

7. Stereomikroskop nach einem der vorhergehenden Ansprüche, **dadurch gekennzeichnet, dass** ein Weisslicht-Korrekturfilter in den Strahlengang der ersten Beleuchtungsquelle (20) bewegbar ist.
